# EUROPEAN PATENT APPLICATION

(11) **EP 2 878 296 A1**
(43) Date of publication of application: **03.06.2015**
(21) Application number: 13005561.9
(22) Date of filing: 29.11.2013
(51) Int. Cl.: A61K 31/165, A61K 31/4015, A61P 25/08

(54) **Pharmaceutical composition comprising lacosamide and levetiracetam**

(71) Applicant: UCB Pharma GmbH, 40789 Monheim (DE)
(72) Inventor: The designation of the inventor has not yet been filed
(74) Representative: Dressen, Frank

(57) **Abstract**

The present application relates to a fixed dose combination comprising lacosamide and levetiracetam, as well as to dosage regimes including such fixed dose combinations. The fixed dose combinations are suitable for the oral or parenteral treatment of various diseases, including epilepsy and/or epileptic seizures.

## Description

### Background of Invention

Lacosamide (LCM, R-2-Acetamido-N-benzyl-3-methoxypropionamide) is approved as adjunctive therapy for partial onset seizures with and without secondary generalization. It is available as IR tablet (50, 100, 150 and 200 mg) for twice daily administration, as oral solution and as i.v. solution, and is commercialized under the tradename Vimpat®.

LCM is effective in a significant amount of epilepsy patients, who are refractory to or insufficiently controlled by other AEDs (Ben-Menachem, et al, Epilepsia, 2007, 48, 1308-1317.)

LCM was found to be particularly effective, if it was given to insufficiently controlled epilepsy patients who were only on one or two previously administered AEDs (Villanova et al, Epilepsy & Behaviour, 29, 2013, 349-356).

The risk for pharmacokinetic or pharmacodynamics drug-drug interaction is low for lacosamide, which has been shown to have no significant effect on plasma levels of other AEDs such as e.g., carbamazepine, levetiracetam, lamotrigine, topiramate, valproate, zonisamide, gabapentin, and phenytoin (Doty et al, Ann Ny Acad Sci, 1291, 2013, 56-68; Patsalos, Clin Pharmacokinet, EPub June 20).

Hence, in order to make use of these favorable properties of LCM and in order to reduce the pill burden of epilepsy patients, a rational dual combination therapy comprising LCM would be helpful.

Hence, the aim of the present disclosure was to provide an effective and safe pharmaceutical composition being a dual (combined) fixed-dose combination ("FDC") comprising LCM, such as, for example, an oral FDC ("oFDC").

WO 2007-144195 discloses that LCM shows beneficial efficacious effects with various AEDs in an animal model of epilepsy. Dual combinations of three different doses of LCM were tested in the 6 Hz epilepsy model with different ED50-adapted doses of each of lamotrigine, carbamazepine, levetiracetam, brivaracetam, topiramate, gabapentin, phenytoin and valproic acid. Based on the isobolographical analyses, lamotrigine, carbamazepine, levetiracetam, brivaracetam, topiramate, gabapentin were considered to be good combination partners from the efficacy and safety perspective. Synergistic effects over all three dose combinations tested were seen for dual combinations of LCM with levetiracetam (LEV, (2R)-2-(2-oxopyrrolidin-1-yl)butanamide), and with carbamazepine but synergistic effects were also identified for two of three dose combinations of LCM with lamotrigine, topiramate and gabapentin. However, the dosages envisaged for these combinations are high in most cases, and were likely to lead to oFDCs with a significant size, making them inconvenient for the patients to swallow.

Doses proposed in WO 2007-144195 for LEV in such combinations with LCM were in the range of 1-3 g/day, doses for gabapentin were even higher (900-3600 g/day), and doses of carbamazepine were 400-1600 g, which was still expected to make the development of a once or twice daily oFDC with a reasonable size very difficult. Hence, taking pharmacological data and proposed doses of WO 2007-144195, as well as galenic aspects into considerations, a combination of LCM with lamotrigine (anticipated dose in the range of about 100-400 mg/day) seems to be an excellent option for an oFDC.

According to a retrospective study of clinical data very recently reported by Villanueva et al (supra), the highest seizure-free rates were achieved when LCM was given to patients who were on valproate, LEV and lamotrigine, while the combination of LCM with LEV was associated with significant fewer withdrawals from treatment because of adverse events than with any other combination of LCM. Accordingly, a combination of LEV+LCM also looks like an attractive option from the pharmacological and clinical point of view.

LEV is presently available as tablet in dosages of 250 mg, 500 mg, 750 mg and 1000 mg to be administered twice daily (commercialized, inter alia under the tradename Keppra®), as i.v. solution, as oral solution, and in the U.S. as extended release tablets of 500 mg and 750 mg, to be administered once daily.

However, the daily amount of LEV usually needed is the gram range. An oFDC of additive size of the presently commercialized tablets of lacosamide and levetiracetam would thus result in an oFDC size inconvenient for the patient to swallow. Instead, the size of a potential oFDC comprising both drugs should not significantly exceed the size of the commercially available, individual Keppra® and Vimpat® tablets.

However, a person skilled in the art could have expected that minimizing the oFDC size by significantly reducing the total amount of excipients, compared to e.g. the commercial lacosamide tablets, could potentially lead to physicochemical LEV-LCM drug-drug interactions. Moreover, certain amounts of excipients are usually needed to control the stability of the formulation and the robustness of the drug release profiles. For example, the amount of excipients in the presently available Vimpat® tablets is well above 50 wt% (see Example 1).

Furthermore, in order to provide an oFDC which can substitute the patient's preexisting LEV and LCM medication, the oFDC should preferably be bioequivalent to the commercial tablet formulations of both, LCM and LEV. Given the complexity of such a combined dual drug formulation, it seemed thus very uncertain or even unlikely that the amount of excipients could be sufficiently reduced while simultaneously meeting the requirements of the above specified target profile of the LEV+LCM oFDC.

### Description of the Invention

It has been found, surprisingly, that LCM and LEV do not adversely physiochemically interact with each other even in the absence of significant amounts of excipients.

Accordingly, the present invention provides pharmaceutical compositions (FDCs) suitable for the joint administration of LCM and LEV, said FDC comprising (a) LCM in an amount of 50 mg-400 mg, and preferably in an amount selected from 50 mg, 100 mg, 150 mg and 200 mg and, (b) LEV in an amount of 250-1500 mg, preferably selected from among 250 mg, 500 mg, 750 mg and 1000 mg.

The FDCs of the present invention can be any LEV-LCM FDC, and are preferably an injectable or infusible solution, or an oral FDC.

Hence, in one aspect, the FDC of the present invention is an oral FDC (oFDC), in particular a solid oFDC. It has been surprisingly found by the inventors of the present application that the amount of excipients can be limited to a low amount such as e.g. below 15 wt% or below 10 wt% thus allowing for a solid oFDC with a small, convenient size, which nevertheless provides a robust release profiles, does not show interactions between LCM and LEV, and provides excellent physical stability of the oFDC, such as e.g. in the case of a tablet, very suitable hardness and friability properties.

In an alternative embodiment of present invention, the FDC is an injectable or infusible FDC (collectively "iFDC").

### Oral Fixed Dose Combinations (oFDC)

In a preferred embodiment of the present invention, the FDC of the present invention is an oFDC for the combined administration of LCM and LEV. Preferably, the oFDC is a solid oFDC, such as a multiple unit dosage form or a single unit dosage form, and particularly preferable a tablet.

The term "solid oFDC" refers to an oFDC in which the amount of solvent/water is below 5 wt%, preferably below 3 wt%, more preferably below 1 wt%.

"Multiple unit dosage forms" comprise powders/particles, pellets, minitablets, or granulates, which may be covered with coatings prior to further processing and/or administration, and/or which may be packed into sachets or capsules. "Multiple unit dosage forms" may be compressed to dispersible tablets consisting of powders/particles, pellets, minitablets, or granulates. Each entity of the "multiple dosing units" (e.g. each pellet, granulate or mini-tablet) is preferably a full functional unit showing in average the in-vitro dissolution properties further defined in this specification".

Single unit dosage forms" are physical entities individually showing the dissolution properties disclosed herein. Upon disintegration single unit dosage forms such as e.g. tablets or dragees, usually do not disperse into separate functional units.

It has been surprisingly found by the inventors of the present application that LCM and LEV do not negatively interact with each other even in high dosages and in the absence of larger amounts of excipients. It was also found, unexpectedly, that a robust release profile of LEV and LCM, and a physically stable formulation can be achieved despite a total drug load of as much as more than 75wt%, more than 80 wt%, more than 85 wt%, and preferably more than 90 wt%, more than 93 wt%, more than 95 wt%, or even more. In one aspect, the drug load is between 90 wt% and 97 wt%, preferably between 93 wt% and 96 wt%. The term "drug load" as used herein refers to the total amount of drug (LEV+LCM) compared to the total weight of the oFDC.

Surprisingly, it has been found that in an oFDC, in particular in a solid oFDC, a certain quantity of levetiracetam works as super-disintegrant which promotes the release of lacosamide, and can substitute for a certain amount of other disintegrants. This is the case, for example, if LEV is present in the solid oFDC in an amount (wt/wt) at least equal to LCM, see Example 14. Further, it has been found, unexpectedly, that LEV in combination with a suitable lubricant, improves the hardness of the solid oFDC, in particular of a fixed dose tablet, such that the use of a binder can be omitted in the solid oFDC.

As a consequence of these findings, the amount of excipients in the solid oFDC could be reduced even further, thus leading to drug loads of more than 90 wt%, more than 91 wt%, more than 92 wt%, more than 93 wt%, more than 94 wt% or even more than 95 wt% of the solid oFDC, while still maintaining the target product profile described further above. In one aspect, the drug load is between 90 wt% and 97 wt%, preferably between 93 wt% and 96 wt%.

Accordingly, one aspect of the present invention relates to a pharmaceutical composition for the combined oral administration of LCM + LEV, said composition comprising at least about 80 wt%,preferably 85 wt%, preferably 90wt% (e.g. between 90 and 97 wt%), more preferably at least about 93wt% (e.g. between 93 and 96 wt%), even more preferably at least about 95wt% of active ingredient, which active ingredient consists of a combination of levetiracetam and lacosamide in a ratio (wt/wt) of about 1:1 to about 20:1, preferably in a rate of 2:1 to about 20:1 and more preferably in a rate of 3:1 to 15:1 or 5:1 to 15:1.

In this application the term "combined administration" shall mean that LEV and LCM are being released from the composition (oFDC) in a coordinated fashion, either sequentially, or more or less simultaneously. For example, in one embodiment the LEV release may start somewhat earlier than the LCM release, or vice versa, but the release of the other active ingredient may start shortly thereafter, thus leading to an overlapping release profile of both compounds from the oFDC. In one embodiment, the release of both compounds may take place sequentially, i.e. first LCM may be released and once finalized, LEV may be released subsequently, or vice versa, as further described herein. In a preferred embodiment, LCM and LCM will be released from the oFDC in an essentially simultaneous immediate release fashion.

In a preferred example at least 85% of both drugs may be released from the oFDC in 15 minutes ("immediate release"). It is also preferred that about 100% of both drugs are dissolved within 30 minutes, or even already in about 20, or already in 15 minutes if measured in vitro using standard dissolution assays as further described herein (see Example 15). Such immediate release dissolution profiles may lead to average maximum plasma (Cmax) levels of LCM after about 1-3 hrs, preferably after 1-2 hrs, and of LEV after about 1-3 hrs, preferably after 1-2 hrs following administration of the immediate-release oFDC to a patient.

Further release profiles of the combined administration are described herein and are also encompassed by the scope of the invention.

The pharmaceutical compositions of the present invention may be given once daily or twice daily, depending on the release profile of LCM and LEV. In a preferred embodiment, the oFDC provides the immediate release of LCM and LEV, and is to be administered twice daily in order to achieve an effective dose of LEV and LCM over the full treatment period, i.e. for about 24 hours.

Suitable dosages of LCM in the pharmaceutical composition (oFDC) may be between 50 and 400 mg, and preferably be selected from 50 mg, 100 mg, 150 mg and 200 mg, whereas preferred LEV dosages may be between 250 mg and 1500 mg and be preferably selected from the group of 250 mg, 500 mg, 750 mg, and 1000 mg. Preferred exemplary dosage combinations of LEV+LCM are shown in **Table 1**:

| **oFDC** | **Example 1** | **Example 2** | **Example 3** | **Example 4** | **Example 5** |
|---|---|---|---|---|---|
| LEV | 500 mg | 500 mg | 750 mg | 750 mg | 1000 mg |
| +LCM | +50 mg | +100 mg | +50 mg | +100 mg | 200 mg |

In one particular embodiment, dosages of LCM in the oFDC are 50 or 100 mg. In one embodiment, preferred dosages of LEV in the oFDC are 500 or 750 mg.

In one embodiment, dosages of LEV+LCM in the oFDC are preferably 50 LCM + 250 mg LEV, 50 mg LCM+ 500 mg LEV; 50 mg LCM+750 mg LEV; 100 mg LCM + 500 mg LEV; 100 mg LCM+750 mg LEV, or 200 mg LCM + 1000 mg LEV.

These oFDCs lead to convenient dosage regimes where patient's "pill burden", i.e. the amount of oFDC units they need to take can be reduced to up to 50%. Suitable exemplary oFDC-saving dosage regimes and the resulting pill burden achieved are given in Table 2 below.

**Table 2: Potential reduction in pill-burden through the use of the LCM/LEV FDC**

| **LCM + LEV Daily dose** | **LCM + LEV; strengths and number of commercially available Vimpat® and Keppra® tablets (twice daily administration)** | **LCM/LEV FDC, corresponding strengths and number of oFDCs (twice daily administration)** | **Reduction in number of medication units^{a}** |
|---|---|---|---|
| 50 mg + 250 mg | 2 x 50 mg + 2x250 mg | 2 x 50/250 mg | 50% |
| 100 mg + 1000 mg | 2 x 50 mg + 2 x 500 mg | 2 x 50/500 mg | 50% |
| 100 mg + 1500 mg | 2 x 50mg + 2 x 750mg | 2 x 50/750 mg | 50% |
| 200 mg + 1000 mg | 2 x 100mg + 2 x 500mg | 2 x 100/500 mg | 50% |
| 200 mg + 1500 mg | 2 x 100mg + 2 x 750mg | 2 x 100/750 mg | 50% |
| 200 mg + 3000 mg | 4 x 50mg + 4 x 750mg | 4 x 50/750 mg | 50% |
| | 2 x 100mg + 4 x 750mg | 4 x 50/750 mg | 33% |
| | 2 x 100mg + 6 x 500mg | 4 x 50/750 mg | 50% |
| 400 mg + 2000 mg | 2 x 200mg + 2 x 1000mg | 2 x 200/1000 mg | 50% |
| | 2 x 200mg + 4 x 500mg | 4 x 100/500 mg | 33% |
| | 4 x 100mg + 4 x 500mg | 4 x 100/500 mg | 50% |
| 400 mg + 3000 mg | 4 x 100mg + 4 x 750mg | 4 x 100/750 mg | 50% |
| | 2 x 200mg + 4 x 750mg | 4 x 100/750 mg | 33% |
| | 2 x 200mg + 6 x 500mg | 4 x 100/750 mg | 50% |
| ^{a} Reduction in number of medication units can also be considered for other doses | | | |

Accordingly, one aspect of the present invention relates to a dosage regime using the oFDCs of the present invention, wherein the number of medications compared to the treatment of single drug units of LEV and LCM is reduced by at least 25%, by at least 33%, or even by 50%.

One aspect of the present invention is a dosing regime with a reduced pill burden compared to existing LEV+LCM combination treatments.

One aspect of the present invention is an improved dosing regime comprising an oral fixed dose combination of levetiracetam and lacosamide, for use as a medicine, preferably for use in the prophylaxis, alleviation and/or treatment of epileptogenesis, of an epileptic disorder and/or of epileptic seizures, which comprises the twice daily administration of one entity of said fixed dosage combination (such as e.g. one tablet or capsule), wherein said one entity provides the combined release of lacosamide and levetiracetam in dosages selected from (a) 50 mg LCM + 250 mg LEV, (b) 50 mg LCM + 500 mg LEV, (c) 50 mg LCM + 750 mg LEV, (d) 100 mg LCM + 500 mg LEV, (e) 100 mg LCM + 750 mg LEV, and (f) 200 mg LCM + 1000 mg LEV. As a consequence, in these particular dosing regimes only two oFDC entities need to be swallowed by the patient per day. Such treatment regimes reduce the pill burden compared to the twice daily immediate release LEV+LCM treatment regime known in the art (i.e. Keppra® and Vimpai® IR tablets) by 50%. In a preferred embodiment, the oFDC entity is a single unit dosage form and particularly preferable a tablet, and even more preferable a tablet which releases LEV and LCM in immediate release fashion.

Hence, one aspect of the present invention is an oral fixed dose combination (oFDC) comprising levetiracetam and lacosamide for use as a medicine, preferably for use in the prophylaxis, alleviation and/or treatment of epileptogenesis, of an epileptic disorder and/or of epileptic seizures, wherein the oral fixed dose combination is a tablet ("fixed dose tablet"), and wherein the prophylaxis, alleviation and/or treatment comprises the twice daily administration of one fixed dose tablet per administration, which fixed dose tablet provides the combined release of lacosamide and levetiracetam in a dosage selected from (a) 50 mg LCM + 250 mg LEV, (b) 50 mg LCM + 500 mg LEV, (c) 50 mg LCM + 750 mg LEV, (d) 100 mg LCM + 500 mg LEV, (e) 100 mg LCM + 750 mg LEV, and (f) 200 mg LCM + 1000 mg LEV. In one aspect, the fixed dose tablet provides the release of both LCM and LEV in the particular dosages described hereinbefore in an immediate release fashion.

One aspect of the present invention is an oral fixed dose combination (oFDC) comprising levetiracetam and lacosamide for use as a medicine, preferably for use in the prophylaxis, alleviation and/or treatment of epileptogenesis, of an epileptic disorder and/or of epileptic seizures, which comprises the twice daily administration of two entities per administration of the fixed dosage combination (such as e.g. tablets or capsules), each entity of which provides the combined release of lacosamide and levetiracetam in dosages selected from (a) 50 mg LCM + 750 mg LEV, (b) 100 mg LCM + 750 mg LEV. Such dosing regimes which include the uptake of 4 oFDCs per day reduces the pill burden compared to the dosing regime available in the art (i.e. Keppra® and Vimpat® IR tablets) by at least 33% (see e.g. Table 2). In a preferred embodiment, the oFDCs are single unit dosage forms, in particular tablets, and even more preferred the oFDCs are tablets which release LEV and LCM in immediate release fashion.

Accordingly, one aspect of the present invention is an oral fixed dose combination comprising levetiracetam and lacosamide for use as a medicine, preferably for use in the prophylaxis, alleviation and/or treatment of epileptogenesis, an epileptic disorder and/or epileptic seizures, wherein the fixed dose combination is a fixed dose tablet, and wherein the prophylaxis, alleviation and/or treatment comprises the twice daily administration of two fixed dose tablets per administration, each of which provides the combined release of lacosamide and levetiracetam in a dosage selected from (a) 50 mg LCM + 750 mg LEV, (b) 100 mg LCM + 750 mg LEV. In one aspect, the fixed dose tablet provides the release of both LCM and LEV in the particular dosages described hereinbefore in an immediate release fashion.

In a preferred embodiment the dosing regimes described herein comprise the administration of two or four oFDC entities per day, which oFDCs comprise identical dosages of LEV+LCM. For example, if a single oFDC entity administered in the morning contains, by way of example, 100 mg LCM + 500 mg LEV, the single oFDC entity to be taken in the evening also contains 100 mg LCM + 500 mg LEV. Likewise, if two oFDC entities administered in the morning each contain 100 mg LCM + 750 mg LEV, then the two oFDC entities to be taken in the evening also preferably each contain 100 mg LCM + 750 mg LEV.

In one aspect the fixed dose tablets of the dosing regimes of the present invention have a drug load of more than 75wt%, more than 80 wt%, more than 85 wt%, more than 90 wt%, more than 93 wt%, more than 95 wt%, or even more. Preferably, the fixed dose tablets of the dosing regimes have a drug load of at least 90wt%, at least 93 wt%, or even of 95 wt% or more. In one aspect, the drug load is between 90 wt% and 97 wt%, preferably between 93 wt% and 96 wt%.

The solid oFDC according to the present invention may be a single unit dosage form, such as a tablet, or a multiple unit dosage form, such as a capsule.

Preferably the oFDC is single unit dosage form, and is particularly preferable a tablet.

Both active ingredients, LCM and LEV are preferably included in the same layer/matrix of the solid oFDC. In other words, the solid oFDC, preferably the tablet, comprises a monolayer/single matrix comprising both active ingredients as well as the excipients. As used herein, the terms "layer" and "matrix" are used interchangeably, unless expressly specified otherwise. Preferably both LCM and LEV are released from the joint layer/matrix in immediate release rates as described hereinbefore.

In one embodiment, LCM and LEV may be delivered from a joint matrix in a modified or delayed release course. This may be achieved either by adding modified release polymers to the matrix or by applying a release modifying coating to an immediate release matrix, or by a combination of release modifying components in the matrix and in the coating.

Such a modified or delayed release profile may lead to lower maximum plasma concentrations Cmax of the drugs, a reduction of the respective Cmax/Cmin ratio, an increased time Tmax to reach Cmax, and to potentially reduced side effects. LCM and LEV may be released from the matrix with delayed rates such that, for example, the maximum concentration of LCM in the patient would be reached at a time Tmax which is more than 2 or 3 hours, more than 4 hours, more than 5 hours, or even more than 6 hours after the administration of the oFDC to a patient, and/or such that the maximum concentration of LEV may be reached at a time Tmax which is more than 3, more than 4, more than 5, or after more than 6 hours after such administration.

Examples for suitable release modifying agents, and suitable drug release profiles can be taken from WO2012/084126, or from WO2006/080029. Suitable release modifying agents in the matrix may include hydrophilic polymers (such as e.g. poloxamers, hydroxyethylcellulose, hydroxypropylcellulose (HPC), methylcellulose, carboxymethylcellulose, hydroxypropylmethylcellulose (HPMC), polyvinyl pyrrolidone, polyvinyl alcohols, modified starch, pregelatinized starch, hydroxypropyl starch, sodium hyaluronate, alginic acid, alginate salts, carrageenan, chitosan, guar gum, pectin, xanthan gum, and the like), hydrophobic polymers (such as e.g. C8-C30 monohydric alcohols, monoglycerides, diglycerides, triglycerides, glycerine esters, hydrogenated castor oil, glyceryl behenate, hydrogenated soybean oil, lauroyl macrogolglycerides, stearyl macrogolglycerides, glyceryl palmitostearate, cethyl palmitate, glycerol esters of fatty acids and cetyl alcohol and the like), and inert polymers (such as acrylic resins, cellulose derivatives, vinyl acetate derivatives, and non-water soluble polyesters, preferably selected from the group of polyvinyl acetate, ethylcellulose, hydroxypropylmethylcellulose acetate phthalate, hydroxypropylmethylcellulose acetate succinate, shellac, polymethacrylic acid derivatives, methacrylic acid copolymer type A, methacrylic acid copolymer type B, methacrylic acid copolymer type C, ammonio methacrylate copolymer type A, ammonio methacrylate copolymer type B, neutral ethyl methyl methacrylate copolymer, basic butylated methacrylate copolymer, and the like).

If the oFDC comprises a release controlling layer, this layer may comprise a water-insoluble wax or at least one polymer capable of delaying the release of LEV and/or LCM. For example, the release controlling layer may comprise at least one release delaying polymer which is selected from acrylic resins, cellulose derivatives, or vinyl acetate derivatives. These polymers may be water-soluble or water-insoluble. These polymers are preferably selected from polyvinyl pyrrolidone, polyvinyl acetate, ethylcellulose, hydroxypropylmethylcellulose acetate phthalate, hydroxypropylcellulose, hydroxypropylmethylcellulose acetate succinate, shellac, methacrylic acid copolymer type A, methacrylic acid copolymer type B, methacrylic acid copolymer type C, ammonio methacrylate copolymer type A, ammonio methacrylate copolymer type B, and basic butylated methacrylate copolymer. Alternatively, water-soluble pore-forming agents may be present in the release controlling layer as well. Water-soluble pore-forming agents such as hydroxypropylmethylcellulose, polyethyleneglycol, mono- or disaccharides, and inorganic salts may be embedded within the less soluble release controlling agent(s) and rapidly dissolve in aqueous environment thus opening pores through which LCM and/or LEV are released.

In an alternative embodiment, LEV and LCM are comprised in different layers/matrices of the inventive oFDC. For example, LEV may be embedded in an inner layer/matrix which may be surrounded by an outer layer encompassing LCM, or vice versa. In the case of such a bilayer tablet, both layers may release the respective drug in immediate release or both layers release the respective drugs in modified release fashion, i.e. in essentially the same or in similar dissolution rates. In one embodiment, LEV and LCM may be released from the different layers in different rates, such that e.g. LEV may be released in substantially immediate release dissolution rates whereas LCM dissolves in a modified or delayed release fashion. Likewise, LCM may be embedded in an immediate release outer layer, whereas LEV may be provided in a modified release fashion from an inner matrix comprising release modifying agents. This differentiated release profile may have the advantage that the peaks of the maximum and minimal drug concentrations of both drugs in the patient (Cmax, Cmin, respectively) do not appear simultaneously, and as a consequence, neither the anticonvulsant effects nor the unwanted effects of both drugs may occur at the same time but are spread over the administration interval. Also, such differing release rates may be used to adapt the oFDC to the lower half-life of LEV compared to LCM, e.g. by delaying the release of LEV while releasing LCM in an immediate release fashion, or less delayed than LEV.

Alternatively, LEV and LCM may be formulated in a multiplicity of multiple unit dosage forms, such as minitablets or granules with the same or with different release profile. For example, LEV may be formulated as immediate release minitablet, while LCM may be formulated as delayed or controlled release minitablets, or vice versa, or LCM and LEV may both be formulated as delayed or modified release minitablets but with a different release rate, and both the LEV and the LCM minitablets may be packed in a joint capsule or sachet for combined administration.

In a preferred embodiment, LEV and LCM are comprised in the same layer of the oFDC, together with the excipients, and both active ingredients release in immediate release mode, i.e. at least 85% of each drug is released within 15 minutes when measured according to the test described in Example 15.

If LEV and LCM are both released from the oFDC in a modified/delayed release fashion, the oFDC may be administered twice a day or once a day. If the oFDCs are administered only once a day, the dosing regimes as described herein are correspondingly adapted.

For example, the improved dosing regime may comprise an oral fixed dose combination of levetiracetam and lacosamide for use as a medicine, preferably for use in the prophylaxis, alleviation and/or treatment of epileptogenesis, of an epileptic disorder and/or of epileptic seizures, which comprises the once daily administration of one or two entities of said fixed dosage combination at a given time (such as e.g. one or two tablets or capsules in the morning), wherein one entity provides the combined release of lacosamide and levetiracetam in dosages selected from e.g. (a) 50 mg LCM + 250 mg LEV, (b) 50 mg LCM + 500 mg LEV, (c) 50 mg LCM + 750 mg LEV, (d) 100 mg LCM + 500 mg LEV, (e) 100 mg LCM + 750 mg LEV, and (f) 200 mg LCM + 1000 mg LEV. As a result, in these particular dosing regimes only one or two oFDC entities need to be swallowed by the patient per day. Such treatment regimes reduce the pill burden compared to the twice daily LEV+LCM treatment regime of the individual LEV and LCM formulations known in the art (2 Vimpat® + 2 Keppra® IR tablets, or 2 Vimpat® + 1 Keppra®XR, extended release tablet) by 33 to 75%. In a preferred embodiment, the oFDC entity for a once-a-day treatment is a single unit dosage form and particularly preferable a tablet.

The pharmaceutical composition (oFDC) according to the present invention, including preferably the fixed dose tablets, comprises excipients in a total amount of up to 25 wt%, preferably up to 20 wt%, up to 15 wt%, up to 10 wt% (e.g. 3-10 wt%), preferably up to 7 wt% (e.g. 4-7 wt%), more preferably up to 5wt%.

Excipients used in the pharmaceutical composition of the present disclosure are principally those known to a person skilled in the art. In particular, the oFDC may comprise glidants, disintegrants, lubricants, stabilizers, antioxidants, binders, fillers, release modifying polymers, coating material, and the like. Suitable excipients may be taken from standard books like e.g. Remington, The Science and Practice of Pharmacy, ed Lippincott, Williams and Wilkins, Baltimore US.

Preferably the amount of excipients is reduced as much as possible, as described herein, and preferably the excipients comprise (a) at least one gildant, (b) at least one disintegrant, (c) at least one lubricant, and optionally (d) a binder or diluent, and/or (e) a release modifying coating.

Hence in one embodiment, the pharmaceutical composition (i.e. the oFDC) according to the invention comprises up to about 15 wt%, up to about 10 wt%, up to about 7 wt%, preferably up to about 5 wt% excipients, wherein the excipients basically consist of (a) at least one gildant, (b) at least one disintegrant, and (c) at least one lubricant, and optionally (d) a release modifying coating.

In one aspect, the glidant is selected from magnesium silicate, magnesium trisilicate, sodium stearate, hydrophobic colloidal silica, magnesium oxide, talc, colloidal silicon dioxide, and is preferably colloidal silicon dioxide, most preferably in an amount of about 0.5-2 wt%. Non limiting examples of suitable qualities of colloidal silicon oxide are for example Aerosil 200, or Aerosil 300 (both available from Degussa Evonik).

In one aspect, the disintegrant is selected from croscarmellose, crospovidone, sodium starch glycolate, pregelatinized starch, native starch, and preferably is crospovidone, particularly in an amount of 2-8 wt%, or 2-5 wt%. Non limiting examples of suitable qualities of crospovidon are for example XL, INF-10 (available from ISP) or Kollidon CL (available from BASF).

In one aspect, the lubricant is selected from magnesium stearate, sodium stearyl fumarate, hydrogenated castor oil, zinc stearate, calcium stearate, sucrose stearate, glycerylpalmitoyl stearate, hydrogenated vegetable oil, potassium benzoate, leucine, polyethylene glycol, palmitic acid, and stearic acid, and is preferably a hydrophilic lubricant selected from sodium stearyl fumarate, sodium laurylsulfate, potassium benzoate, and polyethylene glycol, e.g. in amounts of about 0.75-2 wt%. In a particular embodiment, the hydrophilic lubricant is sodium stearylfumarate, preferably in an amount of about 0.75-2 wt%. If a non-hydrophilic lubricant such as mg stearate is used, the amounts needed are usually somewhat higher any may be at least 1,0 preferably at least 1.2 wt%.

The binder or diluent, if present, may be selected from agar, guar gum, copovidone, povidone, polyethylene glycol, polyethylene oxide, ethylcellulose, methylcellulose, hydroxypropylcellulose, hydroxyethylcellulose, hydroxypropylmethylcellulose, pregelatinized starch, anhydrous lactose and related hydrates, microcrystalline cellulose, powdered cellulose, calcium phosphate tribasic, anhydrous calcium dibasic phosphate and related hydrates, polyols (e.g. mannitol, maltitol, sorbitol), isomalt, sucrose, polydextrose, maltodextrin, dextrate and may be present in an amount of up to 20 wt%, 15 wt%, up to 10 wt%, preferably up to 7.5 wt%, or up to 5 wt%. In one embodiment, the oFDC does not contain any binder or diluent.

In one particular aspect of the invention, the amount of excipients and thus of the volume of the oFDC, preferably the tablet, is limited as far as possible.

In one aspect, the oral pharmaceutical composition (oFDC) is an immediate release composition, preferably a tablet releasing LEV and LCM in an immediate release fashion, comprising
(a) at least 90 wt%, preferably 90-97 wt%, more preferably at least 93 wt% such as 93-96 wt% or even more than 95 wt% of active ingredient, consisting of levetiracetam and lacosamide in a ratio (wt/wt) of about 3:1 to about 20:1, preferably of about 5:1 to about 15:1,
(b) up to 10 wt%, preferably up to 7-10 wt%, preferably up to 7 wt%, such as 4-7 wt%, or only up to 5 wt% of excipients, which comprises at least one disintegrant, preferably in an amount of 2-8 wt%, preferably 2-5 wt%, or 2-3 wt%. Said disintegrant is preferably selected from croscarmellose, crospovidone, sodium starch glycolate, pregelatinized starch, native starch, and preferably is crospovidone.

In one aspect, the pharmaceutical composition (oFDC), preferably a tablet, comprises
(a) at least 90 wt%, preferably 90-97 wt%, more preferably at least 93 wt%, such as 93-96 wt% or even more than 95 wt% of active ingredient, consisting of levetiracetam and lacosamide in a ratio (wt/wt) of about 3:1 to about 20:1, preferably of about 5:1 to about 15:1,
(b) up to 10 wt%, preferably up to 7-10 wt%, preferably up to 7 wt%, such as 4-7 wt%, or only up to 5 wt% of excipients, which comprises
   (b1) 0.5-2 wt%, preferably 0.75-1.25 wt%, such as e.g. about 1 wt% of a glidant, wherein the glidant is preferably selected from magnesium silicate, magnesium trisilicate, sodium stearate, hydrophobic colloidal silica, magnesium oxide, talc, colloidal silicon dioxide,
   (b2) 2-8 wt%, preferably 2-5 wt%, or 2-3 wt% of a disintegrant, wherein the disintegrant is preferably selected from croscarmellose, crospovidone, sodium starch glycolate, pregelatinized starch, native starch, and preferably is crospovidone,
   (b3) 0.75-2.5 wt%, preferably 0.75-2 wt%, more preferably 1-1.5 wt% such as e.g. about 1 wt% of a lubricant, which is preferably selected from magnesium stearate, sodium stearyl fumarate, hydrogenated castor oil, zinc stearate, calcium stearate, sucrose stearate, glycerylpalmitoyl stearate, hydrogenated vegetable oil, potassium benzoate, leucine, polyethylene glycol, palmitic acid, and stearic acid, and more preferably a hydrophilic lubricant selected from sodium stearyl fumarate, sodium laurylsulfate, potassium benzoate, and polyethylene glycol,
wherein such composition preferably releases both, LCM and LEV, in an immediate release fashion.

In one aspect the pharmaceutical composition comprises
(a) at least 93 wt%, preferably at least 95% active ingredient consisting of levetiracetam and lacosamide in a ratio (wt/wt) of about 3:1 to about 20:1, preferably of about 5:1 to about 15:1,
(b) up to 7 wt%, preferably up to 5 wt% of excipients, which comprises
   (b1) 0.5-2 wt%, preferably 0.75-1.25 wt% colloidal silicon dioxide,
   (b2) 2-8 wt%, preferably 2-5 wt% crospovidone, and
   (b3) 0.75-2.5 wt%, preferably 0.75-2 wt% or 1-2 wt%, more preferably 1-1.5 wt% of a hydrophilic lubricant, preferably sodium stearylfumarate,
and wherein such composition preferably releases both, LCM and LEV, in an immediate release fashion. In one aspect, the composition is a modified/delayed composition wherein a release modifying outer coating is added to an immediate release matrix core as described before.

In one aspect, the pharmaceutical composition is a tablet, preferably an immediate release tablet, which comprises an immediate release matrix comprising
(a) at least 93 wt%, preferably at least 95% active ingredient consisting of levetiracetam and lacosamide in a ratio (wt/wt) of about 3:1 to about 20:1, preferably of about 5:1 to about 15:1, wherein
   (a1)the amount of LCM is from 50-400 mg and is preferably selected from 50 mg, 100 mg, 150 mg and 200 mg, and
   (a2) wherein the amount of LEV is from 250-1500 mg, is preferably selected from 250 mg, 500 mg, 750 mg and 1000 mg, and is particularly preferably selected from 250 and 500 mg; and
(b) up to 7 wt%, preferably up to 5 wt% of excipients, comprising
   (b1) 0.5-2 wt%, preferably 0.75-1.25 wt% colloidal silicone dioxide; and
   (b2) 2-5 wt%, preferably 2-3 wt% crospovidone, and
   (b3) 0.75-2.5 wt%, preferably 1-2 wt%, more preferably 1-1.5 wt% of a hydrophilic lubricant, preferably sodium stearylfumarate.

In one aspect, the composition is a modified/delayed composition wherein a release modifying outer coating is added to an immediate release matrix core as described before.

In one specific aspect the pharmaceutical composition is a tablet, preferably an immediate release tablet, which comprises a matrix essentially consisting of
(a) at least 93 wt%, preferably at least 95% active ingredient consisting of levetiracetam and lacosamide in a ratio (wt/wt) of about 5:1 to about 15:1, wherein
   (a1) the amount of LCM is selected from 50 mg, 100 mg, 150 mg, and 200 mg, and
   (a2) wherein the amount of LEV is selected from 250 mg, 500 mg, and 750 mg; and
(b) up to 7 wt%, preferably up to 5 wt% of excipients, comprising
   (b1)0.75-1.25 wt% colloidal silicon dioxide and
   (b2) 2-3 wt% crospovidone, and
   (b3) 1-1.5 wt% of a hydrophilic lubricant, preferably sodium stearylfumarate.

Exemplary formulations with a drug load of 95.3 wt% are given in Table 3 below:

| | | | | | | |
|---|---|---|---|---|---|---|
| Leviteracetam | 250 mg | 500 mg | 500 mg | 750 mg | 750 mg | 1000 mg |
| Lacosamide | 50 mg | 50 mg | 100 mg | 50 mg | 100 mg | 200 mg |
| Colloidal silica 200 | 1.00 % | 1.00 % | 1.00 % | 1.00 % | 1.00 % | 1.00 % |
| Crospovidone | 2.50 % | 2.50 % | 2.50 % | 2.50 % | 2.50 % | 2.50 % |
| Sodium Stearyl Fumarate (SSF) | 1.20 % | 1.20 % | 1.20 % | 1.20 % | 1.20 % | 1.20 % |

Optionally an outer coating layer can be applied to the formulations described above.

One aspect of the present invention is an oral pharmaceutical composition as further described herein, wherein the total volume of said composition is less than the combined total volume of the commercially available Vimpat® and Keppra® formulations at the time of invention, containing the same amounts of LCM and LEV, which volumes are given in the following **Table 4:**

| **Commercial Formulation** | **Drug Load/Dosage** | **Volume (mm³)** |
|---|---|---|
| Vimpat® | 50 mg LCM | X1=99.80 |
| | 100 mg LCM | X2=199.59 |
| | 150 mg LCM | X3=299.39 |
| | 200 mg LCM | X4=399.18 |
| Keppra® | 250 mg LEV | Y1=234.50 |
| | 500 mg LEV | Y2=467.19 |
| | 750 mg LEV | Y3=701.24 |
| | 1000 mg LEV | Y4=949.73 |

By way of example, an oFDC according to the present invention which contains 100 mg LCM and 500 mg of LEV shall preferably have a volume which is less than the combined volume of the Vimpai® and Keppra® tablets having the same drug content, i.e. less than X2 ml + Y2 ml= 199.59 ml + 467.19 ml = less than 666.78 ml.

Even more preferred, the volume of the oFDC should be more than 5%, preferably more than more than 10%, more preferably more than 15%, or even about 20% smaller than the combined volume of the corresponding Vimpat and Keppra tablets having the same dosage.

Exemplary volumes of oFDCs and volume reductions based on the compositions in Table 4 are given in **Table 5:**

| **Doses LEV/LCM** | **oFDC volumes (mm³)** | **Combined volume of commercial Keppra® and Vimpat® formulations** | **% volume reduction by oFDC** |
|---|---|---|---|
| 500/50 | 547.00 | 99.80 + 467.19 = 566.99 | 3.53 |
| 500/100 | 555.06 | 199.59 + 467.19 = 666.78 | 16.76 |
| 750/50 | 727.18 | 99.80 + 701.24 = 801.04 | 9.22 |
| 750/100 | 777.18 | 199.59 + 701.24 = 900.83 | 13.73 |
| 1000/200 | 1088.66 | 949.72 + 399.18 = 1348.90 | 19.29 |

It has been found, surprisingly, that despite the minimized amount of excipients the pharmaceutical compositions show robust dissolution profiles and good physical stability, as shown in the examples.

### Injectable Solutions

Another aspect of the present invention is a fixed dose combination for injection and/or infusion ("iFDC") comprising both, LEV and LCM. Such an iFDC comprises LCM in 50 mg, 100 mg, 150 mg, 200 mg or 400 mg; typical amounts of LEV are 250 mg, 500 mg, 750 mg, 1000 mg or 1500 mg. Said iFDC is either a solution which is ready for use, i.e. for immediate parenteral administration, or is a concentrated solution which may be diluted prior to injection or infusion. Preferably the administration of LCM and LEV from the iFDC takes place simultaneously, i.e. during injection or infusion both drugs are administered as a mixture in respective amounts over time which correspond to the ratio LCM: LEV in the iFDC. Typically, the iFDCs of the present disclosure are immediate release iFDCs which rapidly release levetiracetam and lacosamide into the patient's circulation. Such an iFDC is particularly suitable in emergency situations such as e.g. severe generalized tonic-clonic seizures, acute repetitive seizures or status epilepticus, in particular if these were already resistant to one or more other anticonvulsants.

Due to the excellent physicochemical compatibility between LCM and LEV, inventors found that an iFDC with a rather low volume can be provided which can conveniently be stored in a suitably small container, such as e.g. in a syringe, a vial, or an infusion bag. Hence, in a typical aspect of the invention, the iFDC comprising LCM and LEV is a concentrated solution which may need to be diluted before administration by a factor of e.g. 1:5, 1:10. 1:20. 1:30, or 1:50. Typical amounts of LCM in the iFDC are 50 mg, 100 mg, 150 mg, 200 mg or 400 mg; typical amounts of LEV are 250 mg, 500 mg, 750 mg, 1000 mg or 1500 mg. Typical volumes of the concentrated solutions are between about 2.5 and 50 ml, preferably between 5 and 20 ml. Typical volumes for infusion or injection are between 50 and 500 ml, preferably about 50-150 ml, more preferably about 100 ml. Typical ratios of LEV:LCM (wt/wt) are from about 1:1 to about 20:1, and preferably from about 3:1 to about 15:1, and even more preferably between 5:1 and 15:1.

For example, 200 mg LCM and 1000 mg LEV may be stored in 10-20 ml of a suitable solvent which then may be further diluted prior to use to a typical infusion volume of 50-500 ml, preferably to about 50-150 ml, more preferably to about 100 ml. Likewise, 100 mg LCM and 500 mg LEV may be dissolved in 5 to 10 ml solvent and be diluted to 100 ml prior to administration by e.g. infusion. While the concentrated solution will preferably be stored in vials, syringes, or infusion bags, the dilution to the final infusion volume may typically take place in an infusion bag.

Typical administration times of the parenteral administration are between 5 and 20 minutes, more preferably 10-15 minutes per administration, twice daily.

The following **Table 6** illustrates non-limiting example iFDCs:

| **Doses LEV/LCM** | **Exemplary volumes of the concentrated iOFC storage solution** | **Typical infusion volume** |
|---|---|---|
| 250/50 | 5 ml | 50-100 ml |
| 250/100 | 5-10 ml | 100 ml |
| 500/100 | 5-10 ml | 100 ml |
| 500/150 | 5-15 ml | 100 ml |
| 500/200 | 5-20 ml | 100 ml |
| 750/150 | 7.5-15 ml | 100 ml |
| 750/200 | 7.5-20 ml | 100 ml |
| 1000/100 | 10 ml | 100 ml |
| 1000/200 | 10-20 ml | 100 ml |
| 1000/400 | 10-40 ml | 100 ml |

Suitable liquid phases used in the concentrated solutions are aqueous sodium chloride solutions and/or physiological buffers like e.g. phosphate or acetate buffers which are known to those skilled in the art. The concentrated storage solutions may or may not comprise further excipients such as e.g. stabilizers or preservatives. Prior to use, the iFDC may be diluted with isotonic buffer such as e.g. 0.9% sodium chloride solution, lactated Ringer solution, 5% dextrose solution, or the like.

Accordingly, one aspect of the present invention is an iFDC comprising (a) LCM in an amount of 50 mg-400 mg, and preferably in an amount selected from 50 mg, 100 mg, 150 mg and 200 mg and, (b) LEV in an amount of 250-1500 mg, preferably selected from among 250 mg, 500 mg, 750 mg and 1000 mg, for use as a medicine, wherein said use comprises the twice daily parenteral administration of said FDC. In a preferred aspect, the parenteral administration is by injection or by infusion, and particularly preferable by infusion. In a preferred embodiment the injectable or infusible FDC (iFDC) is a concentrated solution with a concentration of LEV of between 2.5 and 10 wt%, and of LCM of between 1 and 5 wt%, preferably of between about 1 and 2 wt%, which may require dilution prior to its administration, preferably by infusion. The iFDC is preferably for use in the prophylaxis, alleviation and/or treatment of epileptogenesis of an epileptic disorder and/or of epileptic seizures.

One aspect of the present invention relates to an FDC comprising (a) LCM in an amount selected from 50 mg, 100 mg, 150 mg and 200 mg, and in a drug load of between 1 and 5 wt%, preferably of between 1 and 2 wt%, and (b) LEV in an amount selected from 250 mg, 500 mg, 750 mg and 1000 mg, and in a drug load of between 2.5 and 10 wt%, and preferably between 5 and 10 wt%, for use as a medicine, preferably for use in the prophylaxis, alleviation and/or treatment of epileptogenesis, of an epileptic disorder and/or of epileptic seizures, in particular in an emergency situation as described further above, wherein said use includes the dilution of said FDC to a final volume of between 50 and 500 ml, preferably about 50-150 ml, more preferably to about 100 ml, within 48 hours, preferably within 24 hours, more preferably between 12, 6 or 3 hours before administration of the FDC to the patient by infusion. In one embodiment, the infusion of the iFDC to the patient will take place in a period of time of between 10 and 20 minutes, preferably in about 15 minutes.

In an alternative embodiment, the iFDC may contain a ready-for-use solution comprising LEV and LCM in a buffer solution at a volume which is ready for infusion, typically between 50 and 500 ml, preferably about 50-150 ml, more preferably about 100 ml. Typical amounts of LCM in the ready-to-use iFDC are 50 mg, 100 mg, 150 mg, 200 mg or 400 mg; typical amounts of LEV are 250 mg, 500 mg, 750 mg, 1000 mg or 1500 mg. Typical buffers are acetate, phosphate, citrate,carbonate,which may or may not include stabilizers, or preservatives such as benzylic acid, phenol, parabens, chlorobutanol, chlorocresol, benzalkonium chloride, thimérosal, phenoxyethanol, if needed.

One aspect of the present invention is thus an FDC comprising (a) LCM in an amount selected from 50 mg, 100 mg, 150 mg and 200 mg, and (b) LEV in an amount selected from 250 mg, 500 mg, 750 mg and 1000 mg, for use as a medicine, preferably for use in the prophylaxis, alleviation and/or treatment of epileptogenesis, of an epileptic disorder and/or of epileptic seizures, wherein said FDC has a volume of about 100 ml, and contains LEV and LCM in a concentration ready for infusion to a patient within a period of between 10 and 20 minutes, preferably in about 15 minutes. Preferably said ready-for-use iFDC is already contained in an infusion bag.

Hence, one aspect of the present invention is a kit comprising (i) an infusion bag and (ii) an FDC contained in said infusion bag, wherein the FDC comprises (a) LCM in an amount selected from 50 mg, 100 mg, 150 mg and 200 mg, and (b) LEV in an amount selected from 250 mg, 500 mg, 750 mg and 1000 mg, for use as a medicine, preferably for use in the prophylaxis, alleviation and/or treatment of epileptogenesis, of an epileptic disorder and/or of epileptic seizures. Preferably, the FDC in the infusion bag has a volume of about 50-500 ml, preferably about 50-150 ml, more preferably about 100 ml, and contains LEV and LCM in a concentration ready for infusion to a patient within a period of between 10 and 20 minutes, preferably within about 15 minutes.

### Therapeutic Application

The FDCs as described herein may be used in any condition in which LCM and LEV can be used.

For example, LCM is effective against epileptic conditions, and is approved for the adjunctive treatment of partial onset seizures with and without secondary generalization. In addition, LCM is in advanced clinical development in the monotherapy of partial onset seizures, and has reported efficacy at least in single cases of primary generalized seizures, such as e.g. PGTCS, as well as in acute repetitive seizures, and status epilepticus. LCM is also useful in the prevention of epileptogenesis following brain insults such as e.g. caused by status epilepticus, see e.g. WO 2007/14419.

LCM has also been reported to show activity in various preclinical models of pain such as cancer pain, chemotherapy-induced pain, neuropathic pain, including particularly diabetic neuropathic pain, trigeminal neuralgia, atypical face pain, migraine, or muscle pain. Moreover, LCM was active in models of myotonia, dyskinesia, tinnitus, stroke, arthritis, tremor, and psychoses.

LEV is approved for the treatment of partial onset seizures in patients with epilepsy, as adjunctive therapy for myoclonic seizures in patients with Juvenile Myoclonic Epilepsy (JME), and as adjunctive therapy for the treatment of Primary Generalised Tonic-Clonic (PGTC) seizures in patients with Idiopathic Generalised Epilepsy (IGE).

Hence, one aspect of the present invention is an FDC according to the present disclosure for use in the prevention, alleviation and/or treatment of an epileptic condition, pain, migraine, tinnitus, psychoses, and/or myotonia.

In one specific aspect the FDC is for use in the prevention, alleviation and/or treatment of epileptic seizures comprising partial onset seizures with and without secondary generalization, primary generalized epileptic seizures, myoclonic seizures, clonic seizures, tonic seizures, tonic-clonic seizures, atonic seizures, acute repetitive seizures, and absence seizures. In a preferred aspect, the FDC is for use in the prevention, alleviation and/or treatment of epileptic seizures comprising partial onset seizures with and without secondary generalization, primary generalized epileptic seizures, including in particular myoclonic seizures, clonic seizures, tonic seizures, tonic-clonic seizures, and acute repetitive seizures. In one aspect, the FDC is for use in the treatment, alleviation and/or prevention of epileptogenesis following brain insults, such as e.g. injuries, stroke, intoxication, or status epilepticus.

The pharmaceutical compositions (FDCs) of the present invention can be produced according to various methods known to persons skilled in the art.

In a preferred embodiment, if the FDC is a tablet, the manufacturing process is dry granulation. For example, all excipients and the active ingredients may be mixed, sieved, and lubricated, and the resulting mixture compressed and processed to the final dosage form, e.g. tableted. The lubricant may be added to a pre-mixture of the active ingredients and the other excipients (e.g. disintegrant, glider and optionally binder). The compression process may be performed using a roller compactor. Compression forces are typically selected between about 1000 and 3000 DaN such tablets with a hardness of around 100N are produced.

The invention is further illustrated by the following, non-limiting examples.

### Examples

### Comparative Example 1

The composition of the commercially available Vimpat® tablets are shown in Table 7. As can be seen from these examples, the drug load of the presently commercially available LCM tablets is about 40 wt%, while the amount of excipients in the core is more than 50 wt%.

**Table 7:**

| **Component** | **50 mg [mg]** | **100 mg [mg]** | **150 mg [mg]** | **200 mg [mg]** |
|---|---|---|---|---|
| Lacosamide | 50.00 | 100.00 | 150.00 | 200.00 |
| Cellulose, microcrystalline | 45.30 | 90.60 | 135.90 | 181.20 |
| Hydroxypropylcellulose | 13.50 | 27.00 | 40.50 | 54.00 |
| Crospovidone | 10.00 | 20.00 | 30.00 | 40.00 |
| Magnesium stearate | 1.20 | 2.40 | 3.60 | 4.80 |
| Total (tablet core) | 120.00 | 240.00 | 360.00 | 480.00 |

| Coating: | | | | |
|---|---|---|---|---|
| Total (coating) | 6.00 | 12.00 | 18.00 | 24.00 |
| Total (film-coated tablet) | 126.00 | 252.00 | 378.00 | 504.00 |

### Examples 1-13: Exemplary oFDCs

Exemplary compositions of oFDCs according to the present invention are given in Examples 1-13, see Tables 8a and 8b.

All oFDCs were produced according to the method described in Example 17. The in vitro dissolution rates of LEV and LCM were measured according to the method described in Example 16.

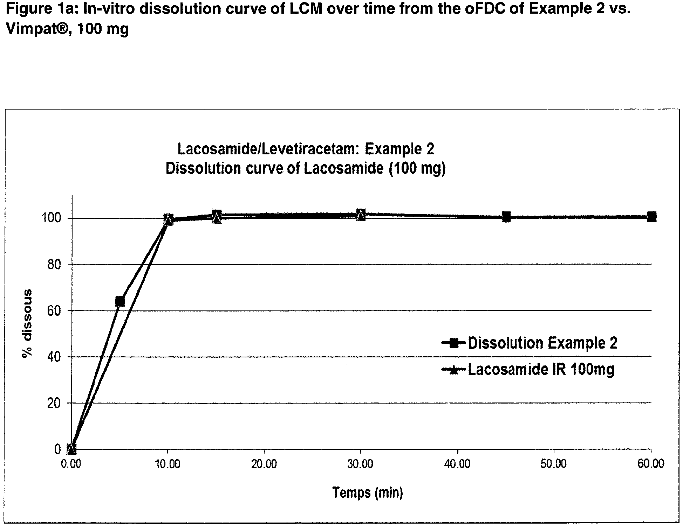

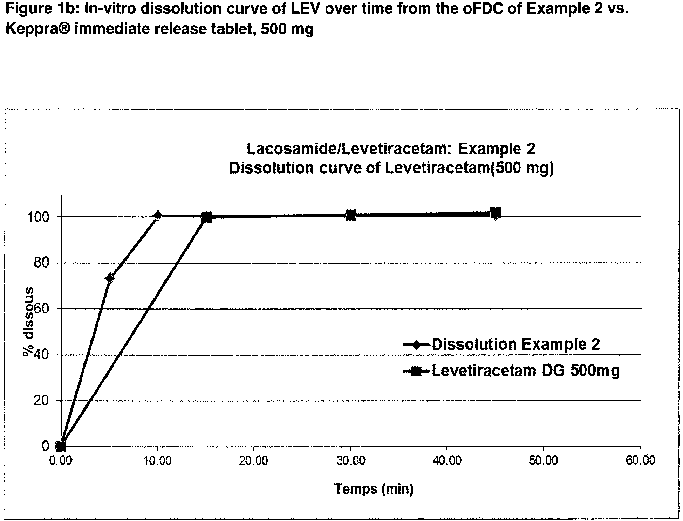

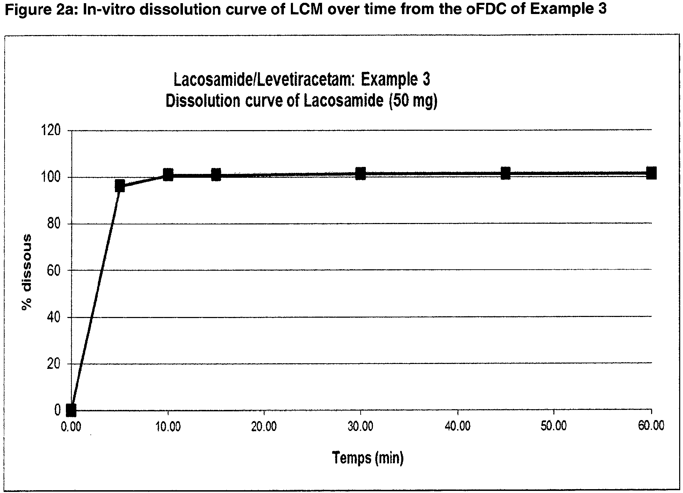

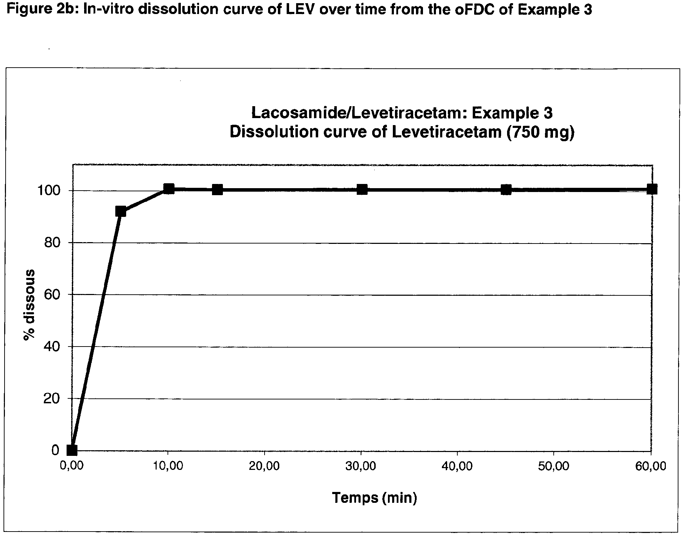

Examples of dissolution profiles are given in Figures 1-2.

**Table 8a:**

| | **Ex-1** | **Ex-2** | **Ex-3** | **Ex-4** | **Ex-5** | **Ex 6** | **Ex-7** | **Ex-8** |
|---|---|---|---|---|---|---|---|---|
| LEV (mg) | 500 | 500 | 750 | 750 | 1000 | 250 | 625 | 250 |
| LCM (mg) | 50 | 100 | 50 | 100 | 200 | 50 | 125 | 200 |
| % Colloidal silica 200 | 1 | 1 | 1 | 1 | 1 | 1 | 1 | 1 |
| % Crospovidone | 2.5 | 2.5 | 2.5 | 2.5 | 5.25 | 5.25 | 5.25 | 5.25 |
| % Sodium Stearyl Fumarate | 1.20 | 1.20 | 1.20 | 1.20 | 1.20 | 1.20 | 1.20 | 1.20 |
| % Dissolution rate LEV | 101 | 101.1 | 100.0 | 100.7 | 101 | 102 | 100 | 102 |
| % Dissolution rate LCM | 98.7 | 102.5 | 106.6 | 107.9 | 103 | 102 | 105 | 99 |
| Friability Rate (%)^{a} | 0.21 | 0.21 | 0.46 | 0.34 | 0.12 | 0.08 | 0.15 | 0.13 |

**Table 8b:**

| | **Function** | **Ex-9** | **Ex-10** | **Ex-11** | **Ex-12** | **Ex-13** |
|---|---|---|---|---|---|---|
| LEV mg | API | 250 | 250 | 250 | 250 | 250 |
| LCM mg | API | 200 | 200 | 200 | 200 | 200 |
| % PEG 600 | Binder | -- | -- | 1 % | -- | -- |
| % Colloidal silica 200 | Glidant | 0.76 | 0.76 | 0.76 | 1 | 1 |
| % Croscarmellose | Disintegrating | -- | 2.6 | 2.1 | | 8 |
| % Crospovidone | Disintegrating | 2.6 | -- | -- | 8 | -- |
| % Mg stearate | Lubricant | -- | -- | 1,4 | -- | -- |
| % Sodium Stearyl Fumarate (SSF) | Lubricant | 1.9 | 1.9 | -- | 1 | 1 |
| % Dissolution rate LEV | | 103 | 97 | 94 | 100 | 111 |
| % Dissolution rate LCM | | 95 | 90 | 89 | 90 | 102 |
| Friability Rate^{a,} | | 0.06 | 0.21 | 0.22 | 0.13 | 0.15 |

| | | | | | | |
|---|---|---|---|---|---|---|
| ^{a}Tablet friability is calculated as the percentage of loss of tablets after 100 rotations. A maximum friability of no more than 1% is considered acceptable. | | | | | | |

### Example 14: LEV as disintegrant to LCM

Table 9 illustrates the surprising effect of LEV on the disintegration time of the tablets. Examples 6 and 14 show a significantly faster disintegration compared to Examples 7 and 8 which have the same composition but the lower content of LEV.

**Table 9:**

| | **Ex-6** | **Ex-14** | **Ex 7** | **Ex 8** |
|---|---|---|---|---|
| LEV (mg) | 1000 | 1000 | 250 | 250 |
| LCM (mg) | 200 | 50 | 200 | 50 |
| % Colloidal silica 200 | 1.00 | 1.00 | 1.00 | 1.00 |
| % Crospovidone | 5.25 | 5.25 | 5.25 | 5.25 |
| % Sodium Stearyl Fumarate (SSF) | 1.20 | 1.20 | 1.20 | 1.20 |
| Tablet disintegration time (min)^{a)b)} | 0.81 | 1.05 | 6.87 | 5.19 |

| | | | | |
|---|---|---|---|---|
| ^{a)} Examples were measured following the compression of the oFDCs with a compression force of 2500 daN. ^{b)} Tablet disintegration time is calculated as an average of 6 individuals. | | | | |

### Example 15: Description of in-vitro dissolution assay

Dissolutions were measured by dissolving the oFDC in 900 ml of 50 mM potassium dihydrogenate phosphate buffer pH 6.8, in USP Type II apparatus (paddle) at 50 RPM with Japanese sinkers, and by measuring the drug release via HPLC.

### Example 16: Description of manufacturing process

At first, the anhydrous colloidal silica and the disintegrating agent (e.g. croscarmellose or crospovidone) were mixed together and passed through a sieve of 1.0 mm. Then the mixture was added to LEV and LCM in a planetary mixer [Collette MP45] and mixed during 20 minutes at speed 1. The lubricant was then added to the blend and mixed for 3 minutes at speed 1. The blend was transferred to a roller compactor [Minipactor Gerteis] and compacted at 7kN/cm. The resulting ribbons were passed through a sieve of 1.0 mm and were loaded into a power assisted rotary tabletting press [Courtoy R090] using a tabletting force of about 1000-3000 daN.

### Examples 18+19: Example iFDC

Fixed dose combinations prepared for administration by infusion or by injection may be prepared by conventional techniques using standard excipients suited for parenteral administration which are well known by persons skilled in the art.

Examples of iFDCs which are either ready for use or which are concentrated solutions that need to be diluted prior to administration, respectively, are given in tables 10 and 11 below:

**Table 10: Fixed dose combination (ready to use solution)**

| | **Function** | **100 mL bag (mg)** |
|---|---|---|
| LEV mg | API | 250 |
| LCM mg | API | 200 |
| Sodium Chloride | Tonicity agent | 600 |
| Sodium Acetate.3H₂O | pH adjustment | 164 |
| Acetic acid glacial | pH adjustment | Ad pH 7.0 |
| WFI | Solvent | To 100 mL |

**Table 11: Fixed dose combination (vial)**

| | **Function** | **20 mL vial (mg)** |
|---|---|---|
| LEV mg | API | 250 |
| LCM mg | API | 200 |
| Sodium Chloride | Tonicity agent | 120 |
| Sodium Acetate.3H₂O | pH adjustment | 32.8 |
| Acetic acid glacial | pH adjustment | Ad pH 7.0 |
| WFI | Solvent | To 20 mL |

## Claims

1. Pharmaceutical composition for the combined administration of lacosamide and levetiracetam, said composition comprising lacosamide in an amount of 50 mg-400 mg and levetiracetam in an amount of 250-1500 mg, wherein said composition is selected from
(a) a fixed dose combination for the oral administration of lacosamide and levetiracetam, comprising at least about 80 wt%, preferably at least 85 wt%, more preferably at least 90wt%, more preferably at least about 93wt%, even more preferably at least about 95wt% of active ingredient consisting of levetiracetam and lacosamide in a ratio (wt/wt) of about 1:1 to 20:1, and
(b) a fixed dose combination for the simultaneous injection or infusion of lacosamide and levetiracetam in a ratio (wt/wt) of about 1:1 to 20:1.

2. Pharmaceutical composition according to claim 1, wherein the amount of lacosamide in the formulation is 50 mg, 100 mg, 150 mg and 200 mg, and wherein the amount of levetiracetam is selected from the group of 250, 500, 750 and 1000 mg, and wherein the ratio (wt/wt) of levetiracetam to lacosamide is from about 3:1 to 15:1.

3. Pharmaceutical composition according to any one of the preceding claims, said composition being a solid oral fixed dose combination comprising at least about 90 wt% of active ingredient consisting of levetiracetam and lacosamide, relative to the total weight of the composition.

4. Pharmaceutical composition according to any one of the preceding claims, said composition being a solid oral fixed dose combination, wherein levetiracetam and lacosamide are contained in the same layer/matrix.

5. Pharmaceutical composition according to any one of the preceding claims, said composition being a solid oral fixed dose combination wherein the composition comprises lacosamide and levetiracetam in a total amount of at least 90 wt%, and excipients in a total amount of up to 10 wt%, preferably up to 7 wt%, more preferably up to 5wt%, wherein the excipients comprise at least one gildant, at least one disintegrant, at least one lubricant, and optionally a binder or diluent.

6. Pharmaceutical composition according to any one of the preceding claims, said composition being a solid oral fixed dose combination wherein the composition comprises up to about 7 wt%, preferably up to 5 wt% excipients, consisting of at least one gildant, at least one disintegrant, and at least one lubricant.

7. Pharmaceutical composition according to any one of claims 5-6, wherein the glidant is selected from magnesium silicate, magnesium trisilicate, sodium stearate, hydrophobic colloidal silica, magnesium oxide, talc, and colloidal silicon dioxide, preferably in an amount of 0.5-2 wt%.

8. Pharmaceutical composition according to any one of claims 5-7, wherein the disintegrant is selected from croscarmellose, crospovidone, sodium starch glycolate, pregelatinized starch, native starch, preferably in an amount of 2-8 wt %.

9. Pharmaceutical composition according to any one of claims 7-8, wherein the lubricant is a hydrophilic lubricant selected from sodium stearyl fumarate, sodium laurylsulfate, potassium benzoate, and polyethylene glycol, preferably in an amount of 0.75-2 wt%.

10. Pharmaceutical composition according to any one of the preceding claims, wherein the composition is a tablet.

11. Pharmaceutical composition according to any one of the preceding claims, said composition being a solid oral fixed dose combination wherein the total volume of the FDC is less than the combined total volume Xn + Yn of the commercial Vimpat® and Keppra® formulations containing the same amounts of lacosamide and levetiracetam, which volumes are given in the following table:
| **Commercial Formulation** | **Drug Load/Dosage** | **Volume (mm³)** |
|---|---|---|
| Vimpat® | 50 mg | X1=99.80 |
| | 100 mg | X2=199.59 |
| | 150 mg | X3=299.39 |
| | 200 mg | X4=399.18 |
| Keppra® | 250 mg | Y1=234.50 |
| | 500 mg | Y2=467.19 |
| | 750 mg | Y3=701.24 |
| | 1000 mg | Y4=949.73 |

12. Pharmaceutical composition according to any one of the preceding claims, said composition being a solid oral fixed dose combination and wherein the composition comprises
(a) at least 93% active ingredient consisting of levetiracetam and lacosamide in a ratio (wt/wt) of about 5:1 to about 15:1
(b) up to 7 wt% of excipients, said excipients comprising
(b1) 0.75-2 wt% colloidal silicon dioxide
(b2) 2-5 wt% crospovidone, and
(b3) 0.75-2.5 wt of a hydrophilic lubricant, preferably sodium stearyl fumarate.

13. Pharmaceutical composition according to any one of the preceding claims, said composition being a solid oral fixed dose combination and wherein the composition provides an in-vitro release of each of lacosamide and levetiracetam, in an amount of at least 85% within 15 minutes, when the in-vitro release of lacosamide and levetiracetam is measured in USP type II apparatus (padlde) using Japanese sinkers, in 900 mL of Phosphate buffer, PH 6.8, at 50 rpm.

14. Pharmaceutical composition according to any one of claims 1 or 2, said composition being an injectable or infusible fixed dose combination which comprises lacosamide and levetiracetam in an amount selected from 50 mg, 100 mg, 150 mg and 200 mg, and (b) LEV in an amount selected from 250 mg, 500 mg, 750 mg and 1000 mg, and wherein both drugs are dissolved either (a) in a volume of between 5 and 20 ml which is further diluted to the final injection or infusion volume of between 50 and 500 ml within 48 hours before use, or (b) in a volume of between about 50 to 500 ml which is ready for infusion to a patient.

15. Kit comprising an infusion bag and the pharmaceutical composition of claim 14 which is contained is said bag.

16. Pharmaceutical composition according to one of the previous claims, wherein the composition is for use in the prevention, alleviation and/or treatment of epileptogenesis, of an epileptic disorder and/or of epileptic seizures, said epileptic seizures comprising partial onset seizures with and without secondary generalization, primary generalized epileptic seizures, myoclonic seizures, clonic seizures, tonic seizures, tonic-clonic seizures, atonic seizures, acute repetitive seizures.

17. Method of manufacturing the oral fixed dose combination according to anyone of claims 1 to 13, comprising dry mixing and compacting the ingredients.

18. Oral fixed dose combination comprising levetiracetam and lacosamide, for use as a medicine which use comprises the twice daily administration of
(i) one entity per administration of said fixed dosage combination, wherein said one entity provides the combined release of lacosamide and levetiracetam in dosages selected from (a) 50 mg LCM + 250 mg LEV, (b) 50 mg LCM + 500 mg LEV, (c) 50 mg LCM + 750 mg LEV, (d) 100 mg LCM + 500 mg LEV, (e) 100 mg LCM + 750 mg LEV, and (f) 200 mg LCM + 1000 mg LEV, or
(ii) two entities per administration of the fixed dosage combination, each entity of which provides the combined release of lacosamide and levetiracetam in a dosage selected from (a) 50 mg lacosamide + 750 mg levetiracetam, and (b) 100 mg lacosamide + 750 mg levetiracetam.

19. Oral fixed dose combination according to claim 18 which is a tablet.

20. Oral fixed dose combination according to any one of claims 18-19, wherein the oral fixed dose combination provides an in-vitro release of each of lacosamide and levetiracetam, in an amount of at least 85% within 15 minutes, when the in-vitro release of lacosamide and levetiracetam is measured in USP type II apparatus (padlde) using Japanese sinkers, in 900 mL of Phosphate buffer, PH 6.8, at 50 rpm.

21. Oral fixed dose combination according to any one of claims 18-20, wherein the total amount of lacosamide and levetiracetam in said oral fixed dose combination is at least 90 wt%.
